# EUROPEAN PATENT SPECIFICATION

(11) **EP 1 747 277 B1**
(45) Date of publication and mention of the grant of the patent: **10.08.2011**
(21) Application number: 04727472.5
(22) Date of filing: 14.04.2004
(51) Int. Cl.: C12N 15/866, A61K 48/00, A61P 25/00

(54) **GENE DELIVERY TO NEURONAL CELLS**
GENZUFÜHRUNG AN NEURONALE ZELLEN
TRANSFERT DE GENES DANS DES CELLULES NEURONALES

(43) Date of publication of application: 31.01.2007
(73) Proprietor: Agency for Science, Technology and Research, Singapore 138668 (SG)
(72) Inventor: WANG, Shu, Singapore 129788 (SG); LI, Ying, Singapore 120703 (SG)
(74) Representative: Blance, Stephen John
(86) International application number: PCT/SG2004/000089
(87) International publication number: WO 2005/100577

(56) References cited:
- US-A1- 2002 193 335
- SARKIS C. ET AL: 'Efficient transduction of neural cells in vitro and in vivo by a baculovirus-derived vector' PROC.NATL.ACAD.SCI. USA vol. 97, no. 26, 1994, pages 14638 - 14643, XP001058902
- ELAZAR M. ET AL: 'Targeting of an expressed neurtoxin by its recombinant baculovirus' THE JOURNAL OF EXPERIMENTAL BIOLOGY vol. 204, 2001, pages 2637 - 2645, XP003003474

## Description

### FIELD OF THE INVENTION

The present invention relates to compositions for gene delivery to neuronal cells.

### BACKGROUND OF THE INVENTION

The use of baculovirus *Auto grapha californica* multiple nuclear polyhedrosis viruses as mammalian cell expression vectors has recently been viewed as an emerging strategy with great promise as a new generation of gene therapy vehicles (Ghosh et aL, 2002; Kost and Condreay, 2002). Baculoviruses display a broad tropism in both proliferating and non-proliferating, quiescent mammalian cells, do not replicate in vertebrate cells and produce little to no microscopically observable cytotoxcity. Baculovirus vector-mediated gene transfection has been investigated in the nervous system. An initial report described an efficient transduction of neural cells *in vitro* and *in vivo* by baculoviral vectors containing a CMV promoter (Sarkis et al., 2000). In primary cell cultures of human embryonic brains, neuroepithelial, neuroblastic, and glial cells could be infected. In vivo studies using adult nude mice demonstrated that mainly astrocytes, with only a few neurons, were transduced in baculovirus vector injected brains. In another study aiming to examine the cell-type specificity of baculovirus-mediated gene expression in the brain (Lehtolainen et al., 2002), cuboidal epithelial cells of the choroids plexus were identified as the main target cells of the viruses in the rat brain, modest gene expression being detected in endothelial cells but very limited or no expression found in other types of brain cells, including neurons and astrocytes.

Major functional cells in the nervous system are neurons, the type of cells that carries out the fundamental task of the system in receiving, conducting and transmitting signals. Therapeutic protection of these cells is one of the main goals of molecular therapy of neurological disorders. Examples include protecting dopaminergic nigrostriatal neurons in Parkinson's disease (Duvoisin, 1992), basal forebrain cholinergic neurons in Alzheimer's disease (Gomez-Isla et al., 1996) and spinal motoneurons in amyotrophic lateral sclerosis. We have previously developed a recombinant baculovirus vector accommodating a strong neuronal specific promoter, CMV E/PDGF, to drive transgene expression (the CMVI E/PDGF described in copending U.S. application No. 10/407,009). After injection into the brain, the baculovirus vector could transduce neurons and mediate transgene expression in neurons nearby the injection site. A persistent transgene expression at relatively high level lasts for at least 4 weeks, much longer than 3 days offered by baculovirus vectors containing a CMV promoter alone.

. The broad anatomical distribution of affected neurons in certain neurological disorders and the relatively inaccessible neuronal location in others present obstacles to effective gene therapy of the disorders. Several virus vectors are capable of overriding the problems by taking advantage of the natural process of axonal transport. This process starts from endocytosis of extracellular substances. In the case of axonal endocytosis, axon terminals are used as ports of entry to take up the substances. The incorporated substances are then transported in axoplasm to the cell body. The viruses that are able to be taken up and transported in this manner include adenovirus (BilangBleuel et al., 1997; Hermens et al., 1997; Choi-Lundberg et al., 1998; Soudais et al., 2001; Peltekian et al., 2002), adeno-associated virus (AAV, Kaspar et al., 2002) and herpes virus (HSV, Breakefield and DeLuca, 1991; Jin et al., 1996). Adenovirus and HSV elicit much stronger immune and inflammatory responses than baculovirus. A single intracerebral injection of either adenovirus or HSV results in dose-dependent inflammatory reactions in the brain, leading to demyelination (McMenamin et al., 1998; Lawrence et al., 1999). AAV vectors, which are able to integrate into the target cell chromosoe, are characterized by the inability to consistently induce immune responses. Their application as gene delivery vectors is however hampered somehow by the small insert size of the viral vectors and the difficulty in manufacturing (Joose and Chirmule, 2003).

### SUMMARY OF THE INVENTION

The invention relates to the following:
- Use of a baculoviral vector in the manufacture of a composition for treating a neuronal disorder,
   wherein the vector is suitable for delivery to a target neuronal cell in a subject, wherein the baculoviral vector is to be administered to a site in the striatum or the intravitreous body of the eye of the subject,
   characterised in that the treatment is to be effected by the delivery of a nucleic acid by the baculoviral vector through axonal transport from the site of administration to the cell body of the target neuronal cell remote from the site of administration;
- A method for tracing an axonal neuronal pathway comprising detecting the transport pathway of a baculovirus adapted for detection that has been administered to a site in the central nervous system of a subject.

Other aspects and features of the present invention will become apparent to those of ordinary skill in the art upon review of the following description of specific embodiments of the invention in conjunction with the accompanying figures.

### BRIEF DESCRIPTION OF THE DRAWINGS

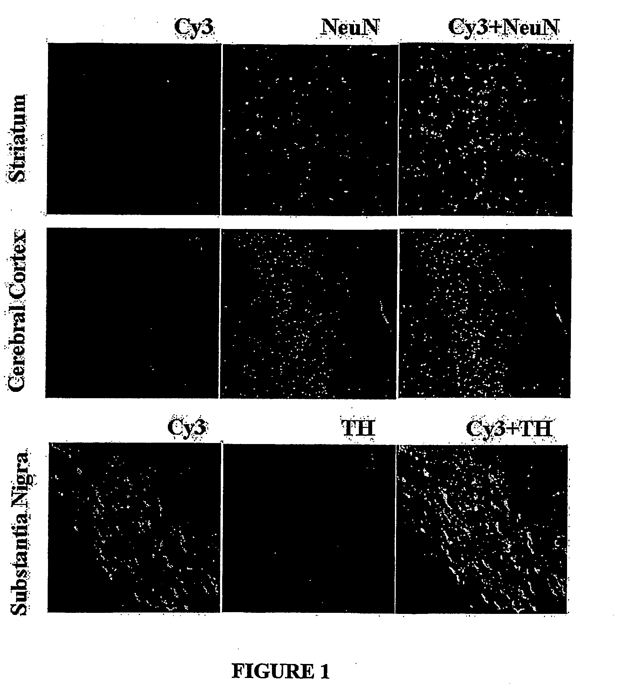

Figure 1 is confocal images of the striatum, the substantia nigra and the cerebral cortex illustrating uptake and transport of Cy3 labeled baculovirus vectors in the rat brain after intra-striatum injection. The scale bar present 50 µm for images of the striatum and the substantia nigra and 100µ m for cerebral cortex;

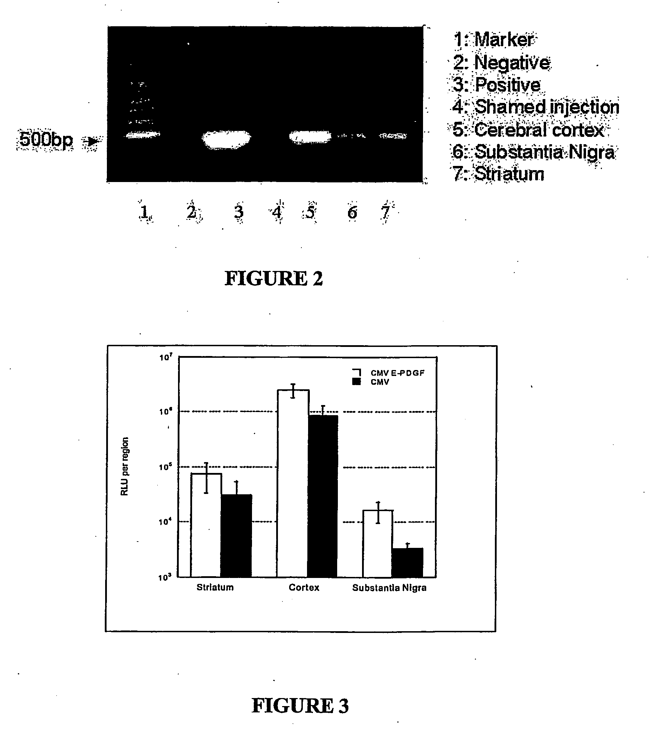

Figure 2 is an image of a PCR analysis of baculovirus DNA after intra-striatum injection. Lane 1, 100bp ladder; lane 2 & lane 4, no injection and mock injection with 1 x PBS as negative control; lane 3, purified viruses as positive control; lanes 5, 6 and 7, tissue samples collected from the cerebral cortex, substantia nigra and striatum, respectively;

Figure 3 illustrates the results of quantitative measurement of luciferase reporter gene activity after intra-striatum injection of baculovirus vectors with the CMV promoter alone or the CMV enchancer/PDGF (CMV E/PDGF) promoter;

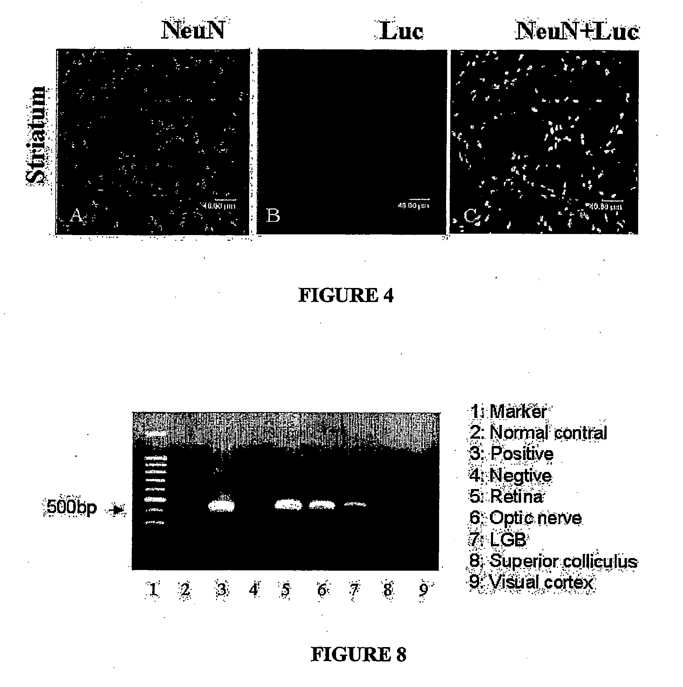

Figure 4 shows confocal images of luciferase expression in the striatum after injection of baculovirus vector with CMV E/PDGF. Samples were double immunostained against neuron-specific nuclear protein (NeuN) to show neurons and against luciferase (Luc) to show luciferase positive cells;

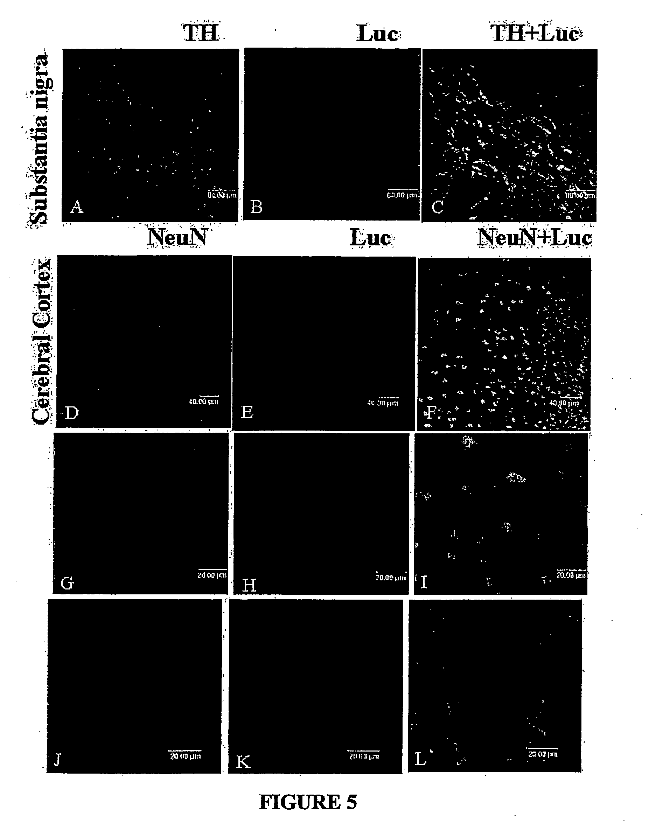

Figure 5 depicts confocal images of luciferase expression in distal regions after intra-striatum injection of baculovirus vector with CMV E/PDGF. A-C: double immunostaining against luciferase to show transfected cells and against tyrosine hydroxylase (TH) to show dopaminergic neurons in the SN; D-L: double immunostaining against luciferase protein and against neuron-specific nuclear protein (NeuN) to show transfected neurons in the cerebral cortex; D-F, low magnification image for the cerebral cortex; G-I, high magnification image showing transfected pyramidal neurons in the cerebral cortex; J-L, high magnification showing untransfected granule cells in the region. Scale bars represent 80 µm for A-C, 40 µm for D-F and 20 µm for G-L;

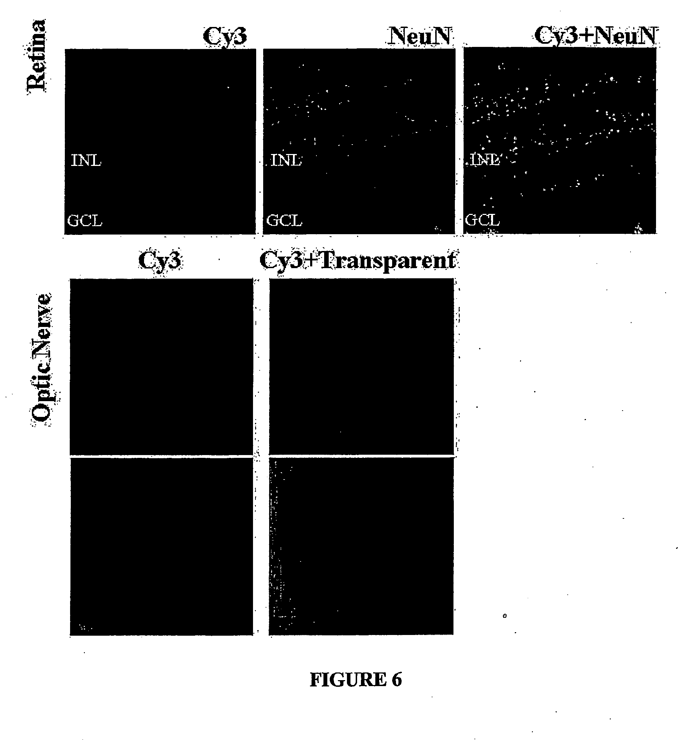

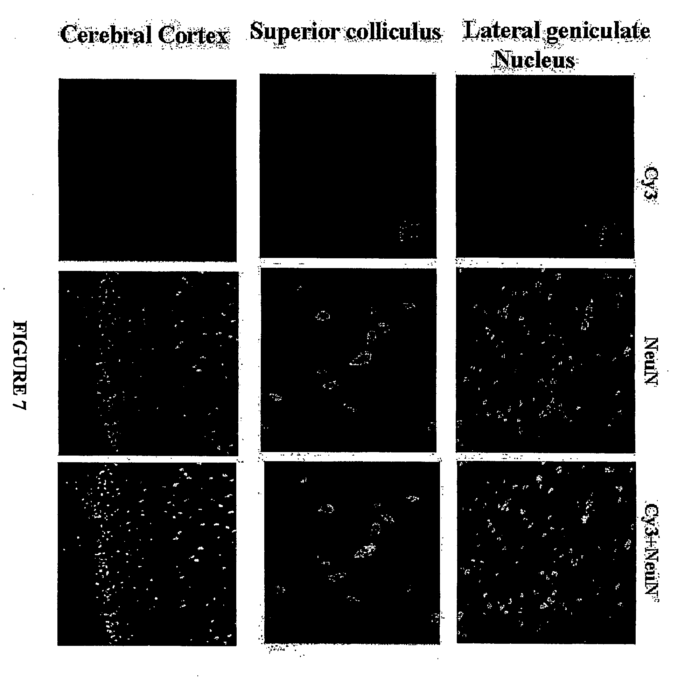

Figure 6 and Figure 7 depict confocal images of uptake and transport of Cy3 labeled baculovirus vectors after intra-vitreous body injection. GCL, ganglion cell layer; INL, inner nuclear layer.

Figure 8 is an image of a PCR analysis of baculovirus DNA after intra-vitreous body injection. Lane 1, 100bp ladder; lane 2 & lane 4, no injection and shamed injection with 1 x PBS as negative control; lane 3, purified viruses as positive control; lanes 5, 6,7,8, and 9, tissue samples collected from retina, optic nerve, lateral geniculate nucleus, superior colliculus, and primary visual cortex, respectively;

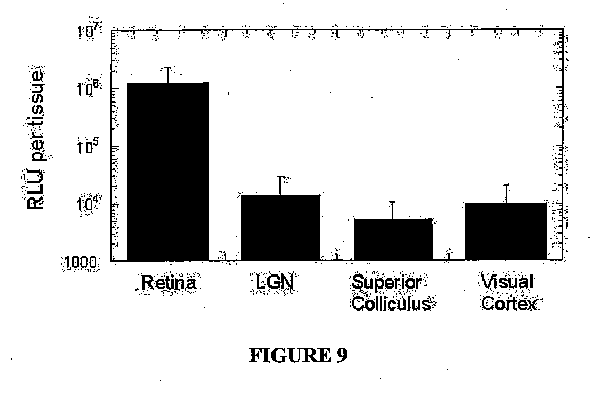

Figure 9 illustrates the results of quantitative measurement of luciferase reporter gene activity after intra-vitreous body injection;

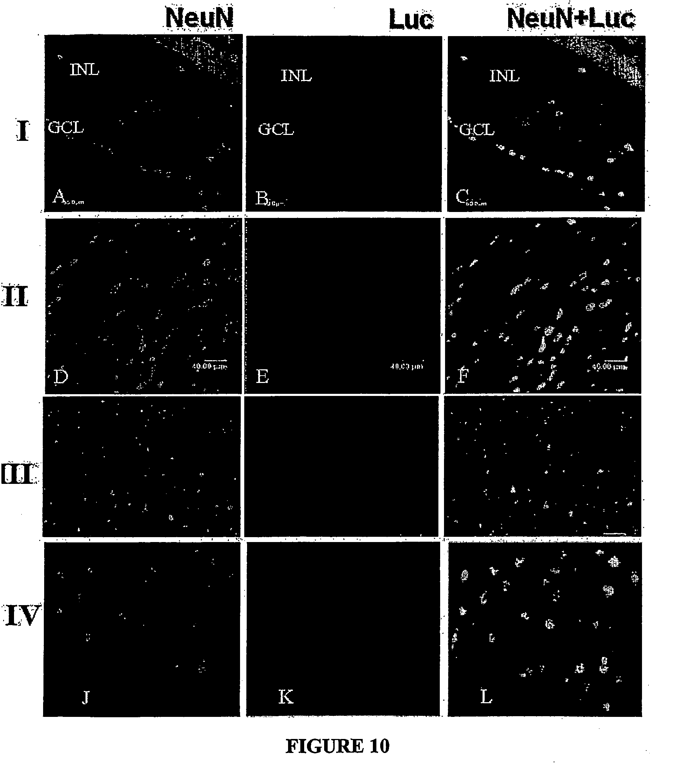

Figure 10 depicts confocal images showing luciferase expression in neurons after intravitreous body injection. Double immunostaining was carried out using antibodies against luciferase to show transfected cells and against neuron-specific nuclear protein (NeuN) to show neurons. Scale bars present 40 µm for A-F, 80 µm for G-I; I: Retina; II: Lateral geniculate body; III: Superior culliculus; IV: Primary visual cortex.

### DETAILED DESCRIPTION

The inventors have demonstrated for the first time that recombinant baculoviral vectors can migrate by axonal transport to neuronal cell bodies, resulting in transgene expression in projection neurons. After stereotaxic injection of Cy3-labeled baculoviral vectors into the rat striatum, retrograde axonal transport of the baculoviral vectors was observed along the corticostriatal pathway and nigrostriatal pathway. After intravitreous body injection, anterograde axonal transport and transsynaptic transport of the virus particles were observed in defined connections of the visual system, from the retina, the optic nerve, the lateral geniculate body, the superior colliculus to the primary visual cortex. PCR analysis confirmed the existence of transported viral DNA in the tissue samples collected from projection fields. Using recombinant baculoviral vectors containing a neuron-specific promoter, transgene expression was detectable in the neuronal cells distal to the injection site.

The use of the axonal transport for neuronal gene transfection allows viral vectors to target neurons at the distal parts of the neural circuits. By using an injection site in a more accessible brain region, the approach can target the populations of neurons deep in the CNS structure or minimize the damage to sensitive regions caused by injection procedure. The striatum is one of the popular regions used to test the approach. There are three major brain regions projecting to striatum: the cerebral cortex, the subtantia nigra, and the intralaminar nuclei of the thalamus. Dopaminergic neurons in the substantia nigra have long axon projecting to large area of the striatum and have been used to study the retrograde transport of many virus vectors, including adenovirus (Ghadge et al., 1995; Peltekian et al., 2002), AAV (Kaspar et al., 2002) and HSV (Jin et al., 1996). The inventors have found, using the baculoviral vector that transgene expression in substantia nigra, although while still highly significant, was relatively lower compared with that in the cerebral cortex, likely due to the difference of the neuron quantity in these two brain regions.

The visual system is a well-defined sensory pathway which possesses both centripetal and centrifugal nonreciprocal connections (Peters, 1985). In rodents, most projections from the retina go contralaterally to the thalamus, to the superior colliculus (SC), and to several structures in the hypothalamus (Millhouse, 1977). Within the thalamus, several specific thalamic nuclei, including the dorsal lateral geniculate nucleus (dLGN), are primary retinorecipient areas. From the dLGN, neuronal projections are relayed to the primary and secondary visual cortices (areas 17 and 18) (Hughes, 1977; Caviness, Jr. and Frost, 1 983). The neurons are further projecting from the visual cortex to many other cortical and subcortical regions, such as the temporal, frontal, and ventrolateral orbital cortices and the basal ganglia. These connections between the retina and the visual cortexes are therefore useful for analyzing transneuronal transport. The transgene expression under a neuron-specific promoter in the retina was confined in ganglion cells after intravitreous body injection of the baculoviral vectors, although the viruses entered both neurons and glia in the retina. Further, intraocular inoculation of baculoviru resulted in a sequential infection of neurons along the structures associated with vision, indicating that the virus can cross several synapses.

A baculoviral vector therefore may be delivered to any target neuronal cell that cannot readily be locally injected, for example, because it is in an area of the central nervous system that is not readily accessible or it is widely distributed in various regions of the central nervous system requiring multiple local injections. By identifying a site in the central nervous system which is remote from such target neuronal cell and administering the vector to the site, the vector may be delivered to the target neuronal cell by axonal transport.

The terms "neuronal cell" and "neuron", which are used interchangeably herein, in accordance with the usual meaning in the art, refer to any conducting cell of the nervous system, which typically includes the cell body, several dendrites and the axon. The terms include a single cell as well as a plurality or population of cells unless the context clearly indicates otherwise.

As will be understood by a skilled person, many neuronal cells have long axons projecting to areas in the central nervous system that are accessible by injection. Therefore, the term target neuronal cell, as used herein, includes the cell body of such a neuronal cell which is remote from its axon that is accessible by injection, as well as another neuronal cell to which the vector may be delivered by trans-synaptic transport. A site is "remote" from the target neuronal cell if the vector injected into the site is transported by axonal transport prior to entering the cell body of the projection neuronal cell or another neuronal cell by trans-syanaptic transport. In contrast, if a vector can be delivered to neuronal cells in and around the area of local injection absent any axonal transport, the site of the local injection is not remote from those neuronal cells. A skilled person can readily identify desired target neuronal cells and site of administration that is accessible by injection and remote from the target neuronal cell such that the vector is delivered to the target neuronal cell by axonal transport. Examples of such target neuronal cells and their remote sites of administration include·target neuronal cells in the optic nerve, retinorecipient areas in the thalamus, such as the dorsal lateral geniculate nucleus and the superior colliculus, as well as the primary and secondary visual cortices; which are accessible from the remote site of the retina or by injection into the vitreous body. Target neuronal cells on the corticostriatal pathway and the nigrostiatal pathway, in the cerebral cortex and substantia nigra, respectively, are accessible by injection at the remote site of the striatum. Further examples of target cells and remote sites by which such cells may be accessed can be determined by reference to various neuroanatomy atlases and texts, for example, Atlas of Functional Neuroanatomy, W. J. Hendleman, CRC Press, 2000; Neuroanatomy: A Functional Atlas of Parts and Pathways, R. Poritsky, Hanley & Belfus, 1992; Functional Neuroanatomy; A. K. Afifi and R. A. Bergman, McGraw-Hill, 1998; Functional Systems: 3D Reconstructions with correlated Neuroimaging, H-J Kretschmann and W. Weinrich, Thieme, 1998; and Neuroanatomy: An Atlas of Structures, Sections and Systems 5th Ed., D. E. Haines, Lippincott, Williams & Wilkins, 2002.

Baculoviruses are well known and characterized and baculoviral vectors for mammalian gene transfection, including the use of *Autographa Californica* are known (see for example, Sakis et al PNAS 97(26)). A skilled person can readily construct any suitable baculoviral vector for use in this invention. The baculoviral vector may be a recombinant baculovirus whose genome has been modified to express a transgene operably linked to a neuron specific promoter or a viral promoter such as a CMV promoter or any operably linked promoter/enhancer as discussed below. The baculovirus may be so modified using standard techniques that will be known to a skilled person, such as PCR and molecular cloning techniques. For example, baculovirus can be readily modified using commercially available cloning and expression systems such as the Bac-To-Bac^{™} Baculovirus Expression system (Gibco BRL, Life Technologies, USA).

The term "gene" is used in accordance with its usual definition, to mean an operably linked group of nucleic acid sequences. The term "transgene" refers to a gene which is foreign, such that, for example, reference to expression of a transgene by a baculovirus refers to expression of a gene that is foreign to the baculoviral genome, and includes a therapeutic gene.

The term "therapeutic gene" or "therapeutic transgenes" as used herein is intended to describe broadly any gene the expression of which effects a desired result, for example, treatment, prevention or amelioration of a neuronal disorder.

Preferably, the gene is a therapeutic gene, including any gene having clinical usefulness, such as a gene encoding a gene product or protein that is involved in disease prevention or treatment, or a gene having a cell regulatory effect that is involved in disease prevention or treatment. The gene product should substitute a defective or missing gene product, protein, or cell regulatory effect in the subject, thereby enabling prevention or treatment of a disease or condition in the subject. Therapeutic genes include growth factor genes (which include genes of fibroblast growth factor gene family, nerve growth factor gene family and insulin-like growth factor genes), and antiapoptotic genes (including genes of bcl-2 gene family).

Neurological illnesses such as stroke, epilepsy, head and spinal cord trauma, Parkinson's disease, Huntington's disease, Alzheimer's disease, amyotrophic lateral sclerosis, and many neurogenetic disorders have devastating effects on the individual and result in high social costs associated with chronic care and lost productivity. A common feature of these illnesses is neuronal loss. Many of the aforementioned disorders are related to the absence, malfunction or ineffectiveness of one gene or more and do not respond well to conventional therapeutic means. Gene transfer into the central nervous system (CNS), which involves the delivery and expression of a therapeutic gene, has, therefore, been considered as a potential approach to treatment of these disorders. This approach may mediate expression levels of neurotrophic factors, anti-apoptotic proteins, antioxidant molecules and other therapeutic factors to restore, halt or prevent neuronal degeneration. Gene therapy also offers much hope for the treatment of CNS malignancies.

For example, there are two main gene therapy strategies for Parkinson's disease. The first approach is transfer of genes that encode trophic factors or protective proteins to slow or halt the continuing neurodegenerative process, such as BDNF (brain derived neurotrophic factor), GDNF (glia derived neurotrophic factor), and antiapoptotic genes like bcl-2. The second approach is transfer of genes encoding neurontransmitter-synthesizing on metabolic enzymes to enhance the function of partially degenerated systems, for example, TH (tyrosine hydroxylase), GTPCH (GTP-cyclohydrolase), and AADA (aromatic amino acid decorboxylase) to recover the striatal DA (dopamine) level and tone.

For Huntington's disease, approaches for gene therapy include 1) antisense strategies to inhibit the expression of mutated 1T15 gene, and 2) protective strategies to keep GABAergic degeneration in the caudate, putaman and cerebral cortex. NGF, CNTF (ciliary neurothropic factor) have been shown to provide protective effect on experimental animal model of Huntington's disease.

The unique characteristics of the CNS, the most sophisticated organ in the body, present several obstacles to successful gene therapy within the CNS. These characteristics include limited access to the CNS due to the physical barriers of the skull and the blood-brain barrier, as well as the nature of terminally differentiated neurons and the difficulty of efficiently transfecting them with therapeutic genes. Moreover, the cell types found within the CNS are very diverse, many of which are critical to physiological functions and highly sensitive to any kinds of changes. This requires the development of CNS gene therapy that is restricted to a particular type of CNS cell, thus ensuring therapeutic effects in the desired cells and limiting side effects caused by gene expression in non-target CNS cells.

In different embodiments, the baculoviral vector comprising a promoter operably linked to a therapeutic gene sequence may be delivered to neuronal cells distal to the site of administration. The promoter may be a strong viral promoter such as CMV or a neuronal specific promoter. Specific gene expression in a selected cell type can be achieved by using neuronal cell specific promoters.

The neuronal specific promoter may be any nucleotide sequence that functions to activate transcription of operably linked sequences within neurons or neuronal cells and substantially not in other cells types. A promoter does not substantially activate transcription if the levels of transcription of operably linked sequences in any of those cell types are sufficiently low so as not to affect the physiological functioning of the cell.

Neuronal cell specific promoters may include promoters for neuronal genes such as Synapsin I, Neuron-specific enolase, Neurofilament-L and Neuropeptide Y and promoters specific for particular types of neuronal cells. For example, tyrosine hydroxylase gene promoter (4.8kb 5' UTR) is specific for catecholaminergic and the CNS neurons, dopamine-b-hydroxylase gene promoter is specific for adrenergic and noradrenegic neurons and L7 Purkinje cell protein promoter is specific for retinal rod bipolar neurons. For these and other neuronal cell specific promoters including, D1A dopamine receptor gene promoter, human hypoxanthine phosphoribosyltransferase promoter, SCG10 promoter, Tα1 α-tubulin promoter, aldolase C promoter, beta-tubulin gene promoter, GnRH gene enhancer and promoter, glutamate decarboxylase 65 gene promoter, beta-galactoside alpha 1,2-fucosyltransferase gene promoter, neuronal nicotinic acetylcholine receptor beta3 gene promoter, GABA(A) receptor delta subunit gene promoter, neuron-specific FE65 gene promoter, N-type calcium channel alpha1B subunit gene promoter and microtubule-associated protein 1B gene promoters, see Harrington CA, Lewis EJ, Krzemien D, Chikaraishi DM. Identification and cell type specificity of the tyrosine hydroxylase gene promoter.Nucleic Acids Res 1987, 15:2363-2384; Coker GT 3rd, Vinnedge L, O'Malley KL; Characterization of rat and human tyrosine hydroxylase genes: functional expression of both promoters in neuronal and non-neuronal cell types. Biochem Biophys Res Commun 1988, 157:1341-1347; Banerjee SA, Hoppe P, Brilliant M, Chikaraishi DM. 5' flanking sequences of the rat tyrosine hydroxylase gene target accurate tissue-specific, developmental, and transsynaptic expression in transgenic mice. J Neurosci 1992, 12:4460-4467; Morita S, Kobayashi K, Mizuguchi T, Yamada K, Nagatsu I, Titani K, Fujita K, Hidaka H, Nagatsu T. The 5'-flanking region of the human dopamine beta-hydroxylase gene promotes neuron subtype-specific gene expression in the central nervous system of transgenic mice. Brain Res Mol Brain Res 1993; 17:239-244; Ishiguro H, Kim KT, Joh TH, Kim KS. Neuron-specific expression of the human dopamine beta-hydroxylase gene requires both the cAMP-response element and a silencer region. J Biol Chem 1993; 268:17987-17994; Hoyle GW, Mercer EH, Palmiter RD, Brinster RL. Cell-specific expression from the human dopamine beta-hydroxylase promoter in transgenic mice is controlled via a combination of positive and negative regulatory elements. J Neurosci 1994, 14:2455-2463; Severynse DM, Colapietro AM, Box TL, Caron MG. The human D1A dopamine receptor gene promoter directs expression of a reporter gene to the central nervous system in transgenic mice. Brain Res Mol Brain Res 1995, 30:336-346; Mouradian MM, Minowa MT, Minowa T. Promoter structure of the human gene coding for the D1A dopamine receptor. Adv Neurol 1993, 60:343-345; Stout JT, Chen HY, Brennand J, Caskey CT, Brinster RL. Expression of human HPRT in the central nervous system of transgenic mice. Nature 1985; 317:250-252; Rincon-Limas DE, Geske RS, Xue JJ, Hsu CY, Overbeek PA, Patel PI. 5'-flanking sequences of the human HPRT gene direct neuronal expression in the brain of transgenic mice. J Neurosci Res 1994; 38:259-267; Schwartz ML, Bruce J, Shneidman PS, Schlaepfer WW. Deletion of 3'-untranslated region alters the level of mRNA expression of a neurofilament light subunit transgene. J Biol Chem 1995; 270:26364-9; Forss-Petter S, Danielson PE, Catsicas S, Battenberg E, Price J, Nerenberg M, Sutcliffe JG. Transgenic mice expressing beta-galactosidase in mature neurons under neuron-specific enolase promoter control. Neuron 1990; 5:187-197; Twyman RM, Jones EA. Sequences in the proximal 5' flanking region of the rat neuron-specific enolase (NSE) gene are sufficient for cell type-specific reporter gene expression. J Mol Neurosci 1997; 8:63-73; Andersen JK, Garber DA, Meaney CA, Breakefield XO. Gene transfer into mammalian central nervous system using herpes virus vectors: extended expression of bacterial lacZ in neurons using the neuron-specific enolase promoter. Hum Gene Ther 1992; 3:487-499; Wuenschell CW, Mori N, Anderson DJ. Analysis of SCG10 gene expression in transgenic mice reveals that neural specificity is achieved through selective derepression. Neuron 1990; 4:595-602; Mori N, Stein R, Sigmund O, Anderson DJ. A cell type-preferred silencer element that controls the neural-specific expression of the SCG10 gene. Neuron 1990; 4:583-594; Hoesche C, Sauerwald A, Veh RW, Krippl B, Kilimann MW. The 5'-flanking region of the rat synapsin I gene directs neuron-specific and developmentally regulated reporter gene expression in transgenic mice. J Biol Chem 1993; 268:26494-26502; Kilic E, Hermann DM, Kugler S, Kilic U, Holzmuller H, Schmeer C, Bahr M. Adenovirus-mediated Bcl-X(L) expression using a neuron-specific synapsin-1 promoter protects against disseminated neuronal injury and brain infarction following focal cerebral ischemia in mice. Neurobiol Dis 2002; 11:275-284; Gloster A, Wu W, Speelman A, Weiss S, Causing C, Pozniak C, Reynolds B, Chang E, Toma JG, Miller FD. The T alpha 1 alpha-tubulin promoter specifies gene expression as a function of neuronal growth and regeneration in transgenic mice. J Neurosci 1994; 14:7319-7330; Thomas M, Makeh I, Briand P, Kahn A, Skala H. Determinants of the brain-specific expression of the rat aldolase C gene: ex vivo and in vivo analysis. Eur J Biochem 1993; 218:143-151; Thomas M, Skala H, Kahn A, Tuy FP. Functional dissection of the brain-specific rat aldolase C gene promoter in transgenic mice. Essential role of two GC-rich boxes and an HNF3 binding site. J Biol Chem 1995; 270:20316-20321; Dennis K, Uittenbogaard M, Chiaramello A, Moody SA. Cloning and characterization of the 5'-flanking region of the rat neuron-specific Class III beta-tubulin gene. Gene 2002 294:269-277; Waldbieser GC, Minth CD, Chrisman CL, Dixon JE. Tissue-specific expression of the human neuropeptide Y gene in transgenic mice. Brain Res Mol Brain Res 1992; 14:87-93; Lawson MA, Macconell LA, Kim J, Powl BT, Nelson SB, Mellon PL. Neuron-specific expression in vivo by defined transcription regulatory elements of the GnRH gene. Endocrinology 2002; 143:1404-1412; Wolfe A, Kim HH, Tobet S, Stafford DE, Radovick S. Identification of a discrete promoter region of the human GnRH gene that is sufficient for directing neuron-specific expression: a role for POU homeodomain transcription factors. Mol Endocrinol 2002; 16:435-449; Makinae K, Kobayashi T, Kobayashi T, Shinkawa H, Sakagami H, Kondo H, Tashiro F, Miyazaki J, Obata K, Tamura S, Yanagawa Y. Structure of the mouse glutamate decarboxylase 65 gene and its promoter: preferential expression of its promoter in the GABAergic neurons of transgenic mice. J Neurochem 2000; 75:1429-14371; Hitoshi S, Kusunoki S, Kanazawa I, Tsuji S. Dorsal root ganglia neuron-specific promoter activity of the rabbit beta-galactoside alphal,2-fucosyltransferase gene. J Biol Chem 1999; 274:389-396; Roztocil T, Matter-Sadzinski L, Gomez M, Ballivet M, Matter JM. Functional properties of the neuronal nicotinic acetylcholine receptor beta3 promoter in the developing central nervous system. J.Biol Chem 1998; 273:15131-15137; Luscher B, Hauselmann R, Leitgeb S, Rulicke T, Fritschy JM. Neuronal subtype-specific expression directed by the GABA(A) receptor delta subunit gene promoter/upstream region in transgenic mice and in cultured cells. Brain Res Mol Brain Res 1997; 51:197-211; Zambrano N, De Renzis S, Minopoli G, Faraonio R, Donini V, Scaloni A, Cimino F, Russo T. DNA-binding protein Pur alpha and transcription factor YY1 function as transcription activators of the neuron-specific FE65 gene promoter. Biochem J 1997; 328:293-300; Kim DS, Jung HH, Park SH, Chin H. Isolation and characterization of the 5'-upstream region of the human N-type calcium channel alpha1B subunit gene. Chromosomal localization and promoter analysis. J Biol Chem 1997; 272:5098-5104 and Liu D, Fischer I. Two alternative promoters direct neuron-specific Expression of the rat microtubule-associated protein 1B gene. J Neurosci 1996; 16:5026-5036. Other neuronal specific promoters will be known to persons skilled in the art.

The promoter comprises at least one nucleotide sequence capable of activating neuronal cell specific expression of operably linked sequences and in some embodiments the nucleotide sequence will retain the minimum binding site(s) for transcription factor(s) required for the sequence to act as a promoter. In some embodiments, the vector comprises multiple copies of the same sequence or two or more different nucleotide sequences each of which is effective to activate the transcription activity. For various promoters which may be used, transcription factor binding sites may be known or identified by one of ordinary skill using methods known in the art as described above. Suitable promoter/enhancer constructs may be readily determined by standard expression assays.

Platelet-derived growth factor ß-chain (PDGF ß) promoter (Sasahara M, Fries JW, Raines EW, Gown AM, Westrum LE, Frosch MP, Bonthron DT, Ross R, Collins T. PDGF ß-chain in neurons of the central nervous system, posterior pituitary, and in a transgenic model. Cell 1991; 64:217-227) has been shown to be specific for CNS neuronal cells, including dopaminergic neurons and in one embodiment, the neuronal cell specific promoter is PDGF ß promoter. In a specific embodiment, the neuronal specific promoter is human PDGF ß promoter.

The transcriptional activity of a neuronal specific promoter in some instrances may be weak, providing less than ideal level of expression of therapeutic gene sequences. In various embodiments, the neuronal specific promoter may be operably linked to an enhancer. An "enhancer" is a nucleotide sequence capable of increasing the transcriptional activity of an operably linked promoter and in the case of a neuronal cell specific promoter, of increasing neuronal cell specific transcriptional activity of the promoter.

A first nucleic acid sequence is operably linked with a second nucleic acid sequence when the sequences are placed in a functional relationship. For example, a coding sequence is operably linked to a promoter if the promoter activates the transcription of the coding sequence. Similarly, a neuronal cell specific promoter and a heterologous enhancer are operably linked when the enhancer increases the neuronal cell specific transcription of operably linked sequences. Enhancers may function when separated from promoters and as such, an enhancer may be operably linked to a neuronal cell specific promoter but may not be contiguous. Generally, however, operably linked sequences are contiguous.

In different embodiments, the enhancer may be a heterologous enhancer, meaning a nucleotide sequence which is not naturally operably linked to a neuronal cell specific promoter and which, when so operably linked, increases the neuronal cell specific transcriptional activity of the neuronal cell specific promoter. Reference to increasing the transcriptional activity is meant to refer to any detectable increase in the level of transcription of operably linked sequences compared to the level of the transcription observed with a neuronal cell specific promoter alone, as may be detected in standard transcriptional assays, including using a reporter gene construct as described in the Examples.

The enhancer may be a known strong viral enhancer or promoter element such as Rous sarcoma virus (RSV) promoter (Gorman CM, Merlino GT, Willingham MC, Pastan I, Howard BH. The Rous sarcoma virus long terminal repeat is a strong promoter when introduced into a variety of eukaryotic cells by DNA-mediated transfection. Proc Natl Acad Sci U S A 1982; 79:6777-6781), SV40 promoter (Ghosh PK, Lebowitz P, Frisque RJ, Gluzman Y. Identification of a promoter component involved in positioning the 5' termini of simian virus 40 early mRNAs. Proc Natl Acad Sci U S A 1981; 78:100-104), CMV enhancer or promoter including CMV immediate early (IE) gene enhancer (CMVE enhancer) (Boshart M, Weber F, Jahn G, Dorsch-Hasler K, Fleckenstein B, Schaffner W. A very strong enhancer is located upstream of an immediate early gene of human cytomegalovirus. Cell 1985; 41:521-530; Niwa H, Yamamura H, Miyazaki J. Efficient selection for high-expression transfectants with a novel eukaryotic vector. Gene 1991; 108: 193-200; see also U.S. Patent Nos. 5,849,522 and 5,168,062).

In an embodiment of the present invention, CMVE enhancer is operably linked upstream to PDGF ß promoter. In a further embodiment, CMVE enhancer is operably linked upstream to PDGF ß promoter and the two sequences are contiguous.

Further embodiments of the neuron specific promoter and an enhancer that may be operably linked including allelic variants and derivatives of known promoter and enhancer sequences are described in US Application No. 10/407,009, which is herein fully incorporated by reference.

In various embodiments, such variants and derivatives may be substantially homologous in that it hybiridizes to the known enhancer and promoter sequences under moderately or stringent, conditions. Hybridization to filter-bound sequences under moderately stringent conditions may, for example, be performed in 0.5 M NaHPO₄, 7% sodium dodecyl sulfate (SDS), 1 mM EDTA at 65°C, and washing in 0.2 x SSC/0.1% SDS at 42°C (see Ausubel, et al. (eds), 1989, Current Protocols in Molecular Biology, Vol. 1, Green Publishing Associates, Inc., and John Wiley & Sons, Inc., New York, at p. 2.10.3). Alternatively, hybridization to filter-bound sequences under stringent conditions may, for example, be performed in 0.5 M NaHPO₄, 7% SDS, 1 mM EDTA at 65°C, and washing in 0.1 x SSC/0.1% SDS at 68°C (see Ausubel, *et al.* (eds), 1989, *supra*). Hybridization conditions may be modified in accordance with known methods depending on the sequence of interest (see Tijssen 1993, Laboratory Techniques in Biochemistry and Molecular Biology - Hybridization with Nucleic Acid Probes, Part I, Chapter 2 "Overview of principles of hybridization and the strategy of nucleic acid probe assays", Elsevier, New York). Generally, stringent conditions are selected to be about 5°C lower than the thermal melting point for the specific sequence at a defined ionic strength and pH. Stringent hybridization may for example be in 5 times SSC and 50% formamide at 42 degrees Celcius and washing in a wash buffer consisting of 0.1.times SSC at 65 degrees Celcius. Washes for stringent hybridization may for example be of at least 15 minutes, 30 minutes, 45 minutes, 60 minutes, 75 minutes, 90 minutes, 105 minutes or 120 minutes.

The degree of homology between sequences may also be expressed as a percentage of identity when the sequences are optimally aligned, meaning the occurrence of exact matches between the sequences. Optimal alignment of sequences for comparisons of identity may be conducted using a variety of algorithms, such as the local homology algorithm of Smith and Waterman, 1981, Adv. Appl. Math 2: 482, the homology alignment algorithm of Needleman and Wunsch, 1970, J. Mol. Biol. 48:443, the search for similarity method of Pearson and Lipman, 1988, Proc. Natl. Acad. Sci. USA 85: 2444, and the computerised implementations of these algorithms (such as GAP, BESTFIT, FASTA and TFASTA in the Wisconsin Genetics Software Package, Genetics Computer Group, Madison, WI, U.S.A.). Sequence alignment may also be carried out using the BLAST algorithm, described in Altschul et al., 1990, J. Mol. Biol. 215:403-10 (using the published default settings). Software for performing BLAST analysis may be available through the National Center for Biotechnology Information (through the internet at http://www.ncbi.nlm.nih.gov/). In various embodiments, the variants and derivatives may be greater than 50%, 80% to 100%, at least 80%, at least 90% or at least 95% identical as determined using such algorithms.

The baculoviral vector may be delivered to any vertebrate animal subject. In one embodiment the subject is a human.

To deliver the baculovirus to a target neuronal cell in a particular region of the central nervous system remote from the site of vector administration, it may be administered to the subject using standard methods known in the art and as described in the examples. For example, the vector may be administered to an accessible site, for example, in the striatum, in order to deliver the vector to neurons at a remote or inaccessible site by axonal transport of the baculovirus or to cortical or spinal cord. Since baculovirus can be transported anterogradely and retrogradely, the accessible site may be located upstream or downstream of the target cells on the same neuronal pathway.

Preferably the baculovirus is administered by injection, including stereotaxic injection and injection into the vitreous body of the eye and cortical or spinal cord injections for regeneration of injured spinal cord. For human patients, the stereotactic frame base will be fixed into the skull and the brain with the stereotactic frame base will be imaged using high resolution MRI. Using appropriate stereotactic software, the images will be translated into 3 dimensional coordinates appropriate for the stereotactic frame for targeted injection of vector DNA.

The baculovirus is administered in an amount sufficient to achieve the desired result, for example, expression of therapeutic gene in the target cells.

When administered to a patient, the baculovirus is administered in an amount effective and at the dosages and for sufficient time period to achieve a desired result. For example, the baculovirus may be administered in quantities and dosages necessary to deliver a therapeutic gene the product of which functions to alleviate, improve, mitigate, ameliorate, stabilize, prevent the spread of, slow or delay the progression of or cure a neuronal disease or disorder.

The effective amount to be administered to a patient can vary depending on many factors such as the pharmacodynamic properties of the baculovirus, the mode of administration, the age, health and weight of the subject, the nature and extent of the disorder or disease state, the frequency of the treatment and the type of concurrent treatment, if any, and the virulence and titre of the virus.

One of skill in the art can determine the appropriate amount based on the above factors. The baculovirus may be administered initially in a suitable amount that may be adjusted as required, depending on the clinical response of the patient. The effective amount of baculovirus can be determined empirically and depends on the maximal amount of the baculovirus that can be administered safely. Since baculovirus has little to no cytotoxicity in vertebrates, large doses may be tolerated. However, the amount of baculovirus administered should be the minimal amount that produces the desired result.

In various embodiments, a dose of about 10⁹ plaque forming units ("pfu") is administered to a human patient. In other embodiments, about 10² to about 10⁹ pfu, about 10⁶ to about 10⁹ pfu, about 10² to about 10⁷ pfu, about 10³ to about 10⁶ pfu, or about 10⁴ to about 10⁵ pfu may be administered in a single dose.

Effective amounts of baculovirus can be given repeatedly, depending upon the effect of the initial treatment regimen. Administrations are typically given periodically, while monitoring any response. It will be recognized by a skilled person that lower or higher dosages than those indicated above may be given, according to the administration schedules and routes selected.

To aid in administration, the virus may be formulated as an ingredient in a pharmaceutical composition. The compositions may routinely contain pharmaceutically acceptable concentrations of salt, buffering agents, preservatives and various compatible carriers. For all forms of delivery, the recombinant baculovirus may be formulated in a physiological salt solution.

The proportion and identity of the pharmaceutically acceptable diluent is determined by chosen route of administration, compatibility with a live virus and standard pharmaceutical practice. Generally, the pharmaceutical composition will be formulated with components that will not significantly impair the biological properties of the live virus. Suitable vehicles and diluents are described, for example, in Remington's Pharmaceutical Sciences (Remington's Pharmaceutical Sciences, Mack Publishing Company, Easton, Pa., USA 1985).

Solutions of the recombinant baculovirus may be prepared in a physiologically suitable buffer. Under ordinary conditions of storage and use, these preparations contain a preservative to prevent the growth of microorganisms, but that will not inactivate the live virus. A person skilled in the art would know how to prepare suitable formulations. Conventional procedures and ingredients for the selection and preparation of suitable formulations are described, for example, in Remington's Pharmaceutical Sciences and in The United States Pharmacopeia: The National Formulary (USP 24 NF 19) published in 1999.

The forms of the pharmaceutical composition suitable for injectable use include sterile aqueous solutions or dispersion and sterile powders for the extemporaneous preparation of sterile injectable solutions or dispersions, wherein the term sterile does not extend to the live baculovirus itself that is to be administered. In all cases the form must be sterile and must be fluid to the extent that easy syringability exists.

The delivery of a therapeutic gene to neurons in a remote or inaccessible region of the CNS allows for treatment of neuronal disorders or diseases. For example, using the method of the invention, dopaminergic neurons in the substantia nigra for therapeutic gene delivery may be targeted for the treatment of Parkinson's disease (Bilang-Bleuel et al., 1997). As well, using the method of the invention, therapeutic transgene expression may be achieved in neurons widely distributed in various regions of the cerebral cortex, which cannot feasibly be reached by single local injection, and therefore allow for corticostriatopallidal neuroprotection of neurons affected in Huntington's disease (HD, Mittoux et al., 2002).

A target neuronal cell in the CNS affected by a neuronal disorder is identified, as is a site in the CNS that is remote from the target neuronal cell. The remote site is the site to which baculoviral vector is to be administered; axonal transport results in delivery of vector to the target neuronal cell.

"Treating" a neuronal disorder refers to an approach for obtaining beneficial or desired results, including clinical results. Beneficial or desired clinical results can include, but are not limited to, alleviation or amelioration of one or more symptoms or conditions, diminishment of extent of disease, stabilization of the state of disease, prevention of development of disease, prevention of spread of disease, delay or slowing of disease progression, delay or slowing of disease onset, amelioration or palliation of the disease state, and remission (whether partial or total), whether detectable or undetectable. "Treating" can also mean prolonging survival of a patient beyond that expected in the absence of treatment. "Treating" can also mean inhibiting the progression of disease, slowing the progression of disease temporarily, although more preferably, it involves halting the progression of the disease permanently. As will be understood by a skilled person, results may not be beneficial or desirable if, while improving a specific disease state, the treatment results in adverse effects on the patient treated that outweigh any benefits effected by the treatment.

A neuronal disorder may be, without limitation, stroke, ischemia, epilepsy, head and spinal cord trauma or injury, Parkinson's disease, Huntington's disease, Alzheimer's disease, amyotrophic lateral sclerosis or a neurogenetic disorder.

A "neuronal cell affected by the disorder" refers to any neuronal cell that has a defect or malfunction that is related to, contributes to or is caused by the neuronal disorder and that can be treated by the expression of a therapeutic gene within the neuronal cell.

Certain viruses, such as HSV and psuedorabies virus, can be transported trans-synaptically and have been successfully used as transneuronal tracers (Babic et al., 1994; Blessing et al., 1994; Norgren, Jr. and Lehman, 1998). Due to the present discovery of the efficient trans-synaptical transport of baculoviruses, such viruses have the potential to act as a potent neuronal tracer for tracing both sensory and motory pathways. Compared to the other transneuronal tracer viruses, for which anterograde axonal transport tends to be relatively slower and less efficient than retrograde transport (Mclean et al., 1989; Card et al., 1990; Card et al., 1991; Vann and Atherton, 1991; Norgren, Jr. et al., 1992; Blessing et al., 1994; Sun et al., 1996), baculoviruses spread quite quickly within the axoplasm both anterogradely and retrogradely. For example, in rats it was observed that within 2 days the recombinant baculovirus was transported from the striatum to the cerebral cortex and from the retina to the primary visual cortex. However, due the bi-directionality of baculovirus axonal transport, baculovirus may not be suited for tracing reciprocal pathways.

One embodiment of the invention is a method for tracing an axonal neuronal pathway comprising detecting the transport pathway of a baculovirus adapted for detection that has been administered to a site in the central nervous system of a subject.

The baculovirus may be adapted for detection by any method known in the art such that it is capable of being detected when injected into the CNS of the subject. For example, the baculovirus may be labeled with a detectable marker on the surface of the virus particle, for example, by attaching or conjugating a label, for example to a coat protein. Alternatively, the baculovirus may be labeled by incorporation of a labeled molecule within the virus particle, for example, during viral assembly. The attached label may be a fluorescent dye moleucule, a radio label, a radiopaque material, luminescent nanocrystal quantum-dots or a material that aids in magnetic resonance imaging.

Radiolabels are known in the art, and include well-known medical radionuclides such as Tc-99m, I-123, I-125, In-111, In-113m, Ga-67, or other suitable gamma-emitters. Radiopaque materials are also known and include iodine compounds, barium compounds, gallium compounds, thallium compounds, and the like. Specific examples of radiopaque materials include barium, diatrizoate, ethiodized oil, gallium citrate, iocarmic acid, iocetamic acid, iodamide, iodipamide, iodoxamic acid, iogulamide, iohexol, iopamidol, iopanoic acid, ioprocemic acid, iosefamic acid, ioseric acid, iosulamide meglumine, ios-umetic acid, iotasul, iotetric acid, iothalamic acid, iotroxic acid, ioxaglic acid, ioxotrizoic acid, ipodate, meglumine, metrizamide; metrizoate, propyliodone, and thallous chloride. Materials that can be detected by or that enhance the effects of magnetic resonance include gadolinium, copper, iron, and chromium and may be prepared in the form of a conventional organometallic chelates, which may then be conjugated to the baculovirus.

The baculovirus may also be adopted for detection by genetic modification to express a gene that encodes a protein that can be detected, for example, a fluorescent protein, under control of a neuron specific promoter. For example, the baculovirus may be modified to express green fluorescent protein under control of the CMV E/PDGF promoter as described in the Examples.

The baculovirus may be detected using any conventional method for detection, including for visualizing diagnostic images and will depend on the particular detectable system used. For example, baculovirus genetically modified to express a fluorescent protein may be detected using fluorescence digital imaging microscopy. Other methods include computed tomography (CT), whole body scan such as position emission tomography (PET), magnetic resonance imaging (MRI), and sonography. Skilled artisans will be able to determine the appropriate method for detecting a particular detectable marker.

The baculovirus may be detected or visualized at a suitable time interval after administration of the virus to the subject, so as to allow for the baculovirus to be transported along the neuronal pathway, and to express a detectable product, if necessary. For example, visualization may occur anywhere between 2 and 20 days following administration to the subject of the baculovirus genetically modified to express a fluorescent protein.

### EXAMPLES

We investigated whether baculovirus vectors could be transported in axons, thus mediating gene expression in regions remote to the injection site. Intra-striatum injection followed by examinations in the corticostriatal pathway and the nigrostriatal pathway and intra-vitreous body injection in the visual pathway were used to study retrograde and anterograde axonal transport, respectively. The recombinant baculovirus vectors accommodating the neuron-specific CMV E/PDGF promoter was used to facilitate gene expression in neuronal cell bodies.

**Materials and methods**

***Production of recombinant virus vectors:*** Recombinant baculoviruses were constructed according to the manual of Bac-To-Bac^{™} Baculovirus Expression system (Gibco BRL, Life Technologies, USA). Luciferase cDNA under the CMV E/PDGF, CMV, or PDGF promoter was inserted into the transfer plasmid pFastBac1^{™}. Each promoter was inserted between Not1 & Xba1, and luciferase cDNA was inserted into the pFASTBac1 between Xho1 & HindIII. Recombinant baculovirus vectors were propagated in Sf9 insect cells according to standard methods (O'Reilly et al., 1992). Budded viruses from insect cell culture medium were filtered through a 0.2 µm pore size filter and concentrated by ultracentrifugation at 25,000 g for 60 min. The viral pellet was re-suspended in phosphate-buffered saline (PBS) and infectious titers were determined by plaque assay on Sf9 cells.

***Cy3 labeling of baculovirus:*** Purified viruses were labeled with the carbocyanine dyes Cy3 (Amersham). Baculovirus stocks to be labeled were adjusted to a concentration of 2 x 10¹⁰ particles/ml in sodium carbonate sodium bicarbonate buffer, pH 9.3. Cy3 reagent was previously reconstituted in 1 ml of 01 M sodium carbonate, pH 9.3 (designed to label 1 mg protein to a final molar dye/protein ratio between 4 and 12). 100 µl of virus preparation was used to mix with 200 µl of Cy3 reconstitute. After 30 min at room temperature, labeled virus was purified by centrifugation at 28,000 g for 30 min. The virus pellet was washed in 1 x PBS for 2 times to remove the unreacted Cy3, and then re-suspended in 1x PBS. The purified viruses were aliquoted into single-use vials and stored at 4°C prior to use.

***Animals:*** Adult male Wistar rats (weighing 250-320 g) were used in this study. In the handling and care of all animals, the International Guiding Principles for Animal Research as stipulated by World Heath Organization (1985) and as adopted by the Laboratory Animal Center, National University of Singapore, were followed.

***In Vivo Injection Methods:*** For intra-striatum injection, rats were anaesthetized by an intraperitoneal injection of sodium pentobarbital (60 mg/kg of body weight) and positioned in a stereotaxic instrument with the nose bar set at O (for striatum injection). Virus particles were separately and bilaterally injected into the striatum at 3 injection points [coordinates (from bregma and dura): anterior (A), +1.5 mm, lateral (L), +2.0 mm, ventral (V), -5.0 mm; A, +0.5 mm, L, +3 mm, V, -5.0mm and A, -0.3 mm, L, +3 mm, V, -5.0mm] or at 1 points [A, +0.5 mm, L, +3 mm, V, - 5.0 mm]. For each injection, the injection value was 5 µl or 10 µl containing 5 x 10⁶ pfu or 1 x 10⁷ viral particles. The rate of injection was 0.5 µl/minute and the needle was allowed to remain *in situ* for 5 minutes before being slowly retracted at the end of each injection. For intra-vitreous body injection, the animals were anaesthetized the same way as previously described. A syringe with a needle having a No. 30 bore size was penetrated through the sclera into the vitreous body posterior to the ora seratta, then 10 µl of the vitreous body was slowly sucked out. After that, 10 µl of the virus particles (10⁷ pfu) was then slowly injected back into the posterior chamber.

***Luciferase assay:*** For luciferase activity assays, virus infected rats were sacrificed 2 days after infection by intracardiac perfusion with 0.1 M PBS (pH 7.4) following deep anesthesia. Tissue samples were collected and stored at -80°C until processing. After adding PBS buffer (100 µl PBS per 50 mg tissue), each sample was homogenized by sonication for 10 seconds on ice, and then centrifuged at 13, 000 rpm at 4°C in a microcentrifuge. 10 µl of the supernatant at room temperature was used for the luciferase activity assay employing an assay kit from Promega. Measurements were made in a single-well luminometer (Berthold Lumat LB 9501) for 10 seconds.

***PCR detection of virus genome from tissue samples:*** Virus infected rats were sacrificed 2 days after infection by intracardiac perfusion with 0.1 M PBS (pH 7.4) following deep anesthesia. Tissue samples were collected and homogenized by mincing with a pestle and the virus genome was extracted according to the standard protocol of a DNeasy^{™} Tissue Kit (Qiagen). The primers target to a sequence of around 400 base pairs, overlapped across both the native virus genome and the inserted reporter gene. PCR primers are listed as below:
5' primer: 5'-AT TGC TCA ACA GTA TGG GCA-3' (SEQ ID NO.:1)
3' primer: 5'-CGA AGA AGG AGA ATA GGG TTG-3' (SEQ ID NO.:2)

Amplification cycles consisted of 94 °C 7min, 1 cycle; 94 °C, 45 s, 52 °C 30 s, 72°C, 30 s, 36 cycles and one final extension cycle at 72 °C for 7 min. To exclude contamination of the whole process, a normal rat brain without virus infection was handled in parallel.

***Immunohistochemical analyses:*** Two days after intra-striatum or intra-vitreous body injection with Cy3 labeled or unlabeled baculovirus, rats were sacrificed (n=2). Following deep anesthesia, all rats were perfused first with 0.1M PBS (pH7.4) solution followed by 4% paraformaldehyde in 0.1 M PBS (pH 7.4). After perfusion, the brains or eye balls were removed and post-fixed in the same fixative for 2-4 hours before they were transferred into 0.1 M PBS containing 20% sucrose and kept overnight at 4°C. Frozen coronal sections of each brain were cut at 30µm thickness and stored in 0.1M PBS. Free-floating sections were washed for 20 min in 0.1M PBS at pH 7.4 containing 0.2% Triton X-100, then blocked with 5% normal goat serum in PBS for 1 hour. Frozen sagittal sections of each eyeball were cut at 30 µm thickness and mounted on gelatin-coated slides.

Sections were then incubated overnight with primary antibodies polyclonal anti-luciferase (Promega; dilution 1:150) and monoclonal against neuron-specific nuclear protein (NeuN) (Chemicon International, USA; dilution 1:500). Sections were washed in 0.1 M PBS and further incubated with anti-rabbit IgG conjugated by Tritc (Sigma-Aldrich, Inc., USA; dilution 1:100) and anti-mouse IgG conjugated by Fitc (Sigma-Aldrich; dilution 1:100) for 1 hour. After incubation, sections were washed three times in PBS. They were then collected on gelatin-coated slides, mounted with DAKO fluorescent mounting medium and covered with cover-slips. Control sections were incubated without primary antibodies.

Cy3 labeled viruses were used to show the virus internalization in vivo. For this group, sections were only incubated overnight with monoclonal against neuron-specific nuclear protein (NeuN) (Chemicon International, USA; dilution 1:500) or monoclonal against glial fibrillary acidic protein (GFAP, Chemicon). Sections were washed in 0.1 M PBS and further incubated with anti-mouse IgG conjugated by Fitc (Sigma-Aldrich; dilution 1:100) for 1 hour. Then sections were treated the same as above.

***Visualization of double labeling with confocal scanning microscopy:*** Sections were examined with a Carl Zeiss LSM410 confocal laser scanning microscope. Each section was initially scanned with a 488 nm laser line, and an emission filter BP 510-525, for the detection of Fitc fluorescein; and with a 543 nm laser line, and an emission filter LP 570, for the detection of Tritc fluorescein.

**Results**

***Inltra-striatum injection:*** We first used Cy3 labeled baculovirus vectors to examine the possible retrograde axonal transport of the vectors. The Cy3 labeled vectors were injected with 1 x 10⁹ plaque-forming units (pfu) of concentrated virus particles into the Wista rat striatum by stereotaxic aparatus. Tissue sections along the corticostriatal pathway and the nigrostriatal pathway were collected and immunostained using antibodies against neuronal nuclear protein (NeuN, a neuronal specific marker) to show the neurons in striatum and cerebral cortex or antibodies against tyrosine hydroxylase (TH, a dopaminergic neuronal specific marker) to show dopaminergic neurons in substantia nigra. As Cy3 was conjugated to envelope proteins of the virus, the fluorescin displayed in the tissue would present the virus particles transported together with viral capsids.

Figure 1 shows the confocal images of striatum, cerebral cortex and substantia nigra. In the striatum the Cy3 signals were detected in both NeuN-positive or negative cells, indicating the entry of virus particles to both neuronal and non-neuronal cells. In cerebral cortex and substantia nigra, Cy3 signals were strictly co-localized with either NeuN or TH signals, suggesting that virus particles, after injected to the striatum, entered axon terminals and moved in axoplasm to the somas of projection neurons located in the cerebral cortex and the substantia nigra. This was supported by PCR analysis, which detected baculovirus genome in the cerebral cortex and the substantia nigra remote from the injected striatum (Figure 2).

To test the possibility of gene transfer in projection neurons, the expression of luciferase reporter gene driven by the neuron-specific CMV E/PDGF promoter the viral CMV promoter was examined. Luciferase activity was measured 2 days after injection of 5x10⁶ pfu of recombinant baculovirus. Luciferase activity was detectable not only in the injected striatum but also in distal substantia nigra and cerebral cortex regions (Figure 3). More than 90% of the total luciferase activity from three parts was detected in the cerebral cortex, and about 1% in the substantia nigra.

A further experiment was performed to determine the type of cells with luciferase expression using immunohistological staining. In the striatum, luciferase signals were detected in both NeuN positive and negative cells (Figure 4). As shown in Figure 5, the expression of luciferase reporter gene was strictly co-localized with either NeuN-positive neurons in the cerebral cortex or TH-positive neurons in the substantia nigra. In the cerebral cortex, luciferase signals were detected mainly in large pyramidal neurons located in deeper layers (Figure 5), a group of cells known to project their long axons to the putamen of the striatum. There were very few transduced cells, with granule interneuron morphology, in surface layers of the cerebral cortex (Figure 5).

The luciferase expression in substantia nigra was relatively lower compared with that in the cerebral cortex, although its absolute value was still highly significant. Considering the difference of neuron quantity in these two brain regions, the difference in transgene expression levels is not unexpected.

***Intra-vitreous Body Injection:*** To investigate possible anterograde axonal transport of baculoviruses, Cy3 labeled vectors were injected into the rat vitreous body in order to transduce the retina ganglio cells (RGCs). These neurons have long axons projecting to the lateral geniculate body (LGB) and superior colliculus (SC) and forming synapse with interneurons located there. From the LGB, the axons of the interneurons fan out through the deep white matter of the brain to form the optic radiations, which will ultimately terminate in the primary visual cortex at the posterior part of the brain.

After intra-vitreous body injection of 10⁹ pfu of the labeled baculovirus vectors, virus particles were observable under confocal microscope in different parts of the visual system, from the retina, the optic nerve, the LGB, the SC to the primary visual cortex. In the retina, virus particles were detected in both NeuN-positive and negative cells (Figure 6 and Figure 7). The labeled particles were easily detected in the optic nerve and distributed along the sagittal section of the nerve, indicating the anterograde travel of the virus along the axons of the RGCs. The Cy3 signals were strictly located in NeuN-positive neurons in the LGB and the SC, indicating a possible trans-synaptic transport of the viral particles to post-synaptic neurons. The hypothesis of the transport of virus vectors across the synaptic cleft was strongly supported by the detection of the Cy3 signals in the tertiary neurons in primary visual cortex. PCR analysis of the virus genome in the samples collected from the different parts of the visual system provided another piece of the evidence for the anterograde transport and trans-synaptic transport of the baculovirus vectors (Figure 8).

The possibility of using baculoviruses for gene transfer in projected, post-synaptic neurons was then investigated with a luciferase reporter gene vector under control of the neuron-specific CMV E/PDGF promoter. After intra-vitreous body injection of 1x10⁷ pfu of the recombinant baculovirus, luciferase activity was detected in all parts of the visual circuit, with the highest transgene expression in the retina, followed by that in LGN and primary visual cortex (Figure 9). The SC gave the lowest luciferase expression, consistent with the fact that only a small amount of axons from RGCs projecting to this region in rats (http://thalamus.wustl.edu/course/ and http://psych.athabascau.ca/html/Psych402/Biotutorials/). As revealed by immunohistological staining, luciferase expression was confined only in neurons, no matter which part of the visual system was examined (Figure 10). Most of the transduced neurons in the LGB were large neuron cells, likely to be the large M cells of the LGB that receive the inputs from large ganglion cells.

In rodents, most projections from the retina go contra-laterally to the thalamus, to the superior colliculus (SC) and to several structures in the hypothalamus. Within the thalamus, several specific thalamic nuclei, including the dorsal lateral geniculate nucleus (dLGN), are primary retinorecipient areas. From the dLGN, neuronal projections is relayed to the primary and secondary visual cortices (areas 17 and 18). The neurons further project from the visual cortex to many other cortical and subcortical regions, such as the temporal, frontal, and ventrolateral orbital cortices and the basal ganglia. These connections between the retina and the visual cortexes are therefore useful for analyzing transneuronal transport. Here, we found that, with the assistance of a neuron-specific promoter, the transgene expression in the retina was confined in ganglion cells after intravitreous body injection of recombinant baculovirus vectors, although the viruses entered both neurons and glia in the retina as revealed through Cy3 labeling. This study showed further that intraocular inoculation of baculovirus resulted in a sequential infection of neurons along the structures associated with vision, providing the evidence for the ability of the virus to cross several synapses.

### REFERENCES:

Airenne, KJ, M O Hiltunen, M P Turunen, A M Turunen, O H Laitinen, M S Kulomaa, S Yla-Herttuala, (2000). Baculovirus-mediated periadventitial gene transfer to rabbit carotid artery. Gene Ther., 7: 1499-1504.
Babic, N., Mettenleiter, T. C., Ugolini, G., Flamand, A., & Coulon, P. 1994. Propagation of pseudorabies virus in the nervous system of the mouse after intranasal inoculation. Virology, 204(2): 616-625.
Bei Hui Liu, Xu Wang, Yue Xia Ma, Shu Wang, (2004) CMV Enhancer/Human PDGF-Promoter for Neuron-Specific Transgene Expression. Gene Ther., 11(1) 52-60.
Bilang-Bleuel, A., Revah, F., Colin, P., Locquet, I., Robert, J. J., Mallet, J., & Horellou, P. 1997. Intrastriatal injection of an adenoviral vector expressing glial-cellline-derived neurotrophic factor prevents dopaminergic neuron degeneration and behavioral impairment in a rat model of Parkinson disease. Proc.Natl.Acad.Sci.U.S.A, 94(16):8818-8823.
Blessing, W. W., Ding, Z. Q., Li, Y. W., Gieroba, Z. J., Wilson, A. J., Hallsworth, P. G., & Wesselingh, S. L. 1994. Transneuronal labelling of CNS neurons with herpes simplex virus. Prog.Neurobiol., 44(1): 37-53.
Boyce, FM, N L Bucher, (1996) Baculovirus-mediated gene transfer into mammalian cells. Proc. Natl. Acad. Sci. U.S.A., 93: 2348-2352.
Breakefield, X. O. & DeLuca, N. A. 1991. Herpes simplex virus for gene delivery to neurons. New Biol., 3(3): 203-218.
Card, J. P., Rinaman, L., Schwaber, J. S., Miselis, R. R., Whealy, M. E., Robbins, A. K., & Enquist, L. W. 1990. Neurotropic properties of pseudorabies virus: uptake and transneuronal passage in the rat central nervous system. J.Neurosci, 10(6): 1974-1994.
Card, J. P., Whealy, M. E., Robbins, A. K., Moore, R Y., & Enquist, L. W. 19991. Two alpha-herpesvirus strains are transported differentially in the rodent visual system. Neuron, 6(6): 957-969.
Caviness, V. S., Jr. & Frost, D. O. 1983. Thalamocortical projections in the reeler mutant mouse. J.Comp Neurol, 219(2): 182-202.
Charlton, C. A. & Volkman, L. E. 1993. Penetration of Autographa californica nuclear polyhedrosis virus nucleocapsids into IPLB Sf 21 cells induces actin cable formation. Virology, 197(1): 245-254.
Choi-Lundberg, D. L., Lin, Q., Schallert, T., Crippens, D., Davidson, B. L., Chang, Y. N., Chiang, Y. L., Qian, J., Bardwaj, L., & Bohn, M. C. 1998. Behavioral and cellular protection of rat dopaminergic neurons by an adenoviral vector encoding glial cell line-derived neurotrophic factor. Exp.Neurol., 154(2): 261-275.
Diefenbach, R. J., Miranda-Saksena, M., Diefenbach, E., Holland, D. J., Boadle, R. A., Armati, P. J., & Cunningham, A. L. 2002. Herpes simplex virus tegument protein US11 interacts with conventional kinesin heavy chain. J.Virol., 76(7): 3282-3291.
Duvoisin, R. C. 1992. Overview of Parkinson's disease. Ann.N.Y.Acad.Sci., 648: 187-193.
Dwarakanath, RS; C L Clark, A K McElroy, D H Spector (2001) The use of recombinant baculoviruses for sustained expression of human cytomegalovirus immediate early proteins in fibroblasts. Virology, 284: 297-307.
Ghadge, G. D., Roos, R. P., Kang, U. J., Wollmann, R., Fishman, P. S., Kalynych, A. M., Barr, E., & Leiden, J. M. 1995. CNS gene delivery by retrograde transport of recombinant replication-defective adenoviruses. Gene Ther., 2(2): 132-137.
Ghosh, S., Parvez, M. K., Banerjee, K., Sarin, S. K., & Hasnain, S. E. 2002. Baculovirus as mammalian cell expression vector for gene therapy: an emerging strategy. Mol.Ther., 6(1): 5-11.
Gomez-Isla, T., Price, J. L., McKeel, D. W., Jr., Morris, J. C., Growdon, J. H., & Hyman, B. T. 1996. Profound loss of layer II entorhinal cortex neurons occurs in very mild Alzheimer's disease. J.Neurosci., 16(14): 4491-4500.
Hermens, W. T., Giger, R J., Holtmaat, A. J., Dijkhuizen, P. A., Houweling, D. A., & Verhaagen, J. 1997. Transient gene transfer to neurons and glia: analysis of adenoviral vector performance in the CNS and PNS. J.Neurosci.Methods, 71(1): 85-98.
Hofmann, C, V Sandig, G Jennings, M Rudolph, P Schlag, M Strauss, (1995) Efficient gene transfer into human hepatocytes by baculovirus vectors. Proc. Natl. Acad. Sci. U.S.A., 92: 10099-10103.
Hughes, H. C. 1977. Anatomical and neurobehavioral investigations concerning the thalamo-cortical organization of the rat's visual system. J.Comp Neurol., 175(3): 311-336.
Jin, B. K., Belloni, M., Conti, B., Federoff, H. J., Starr, R., Son, J. H., Baker, H., & Joh, T. H. 1996. Prolonged in vivo gene expression driven by a tyrosine hydroxylase promoter in a defective herpes simplex virus amplicon vector. Hum.Gene Ther., 7(16): 2015-2024.
Joose, K. & Chirmule, N. 2003. Immunity to adenovirus and adeno-associated viral vectors: implications for gene therapy. Gene Ther., 10(11): 955-963.
Kaspar, B. K., Erickson, D., Schaffer, D., Hinh, L., Gage, F. H., & Peterson, D. A. 2002. Targeted retrograde gene delivery for neuronal protection. Mol.Ther., 5(1): 50-56.
Kost, T. A. & Condreay, J. P. 2002. Recombinant baculoviruses as mammalian cell gene-delivery vectors. Trends Biotechnol., 20(4): 173-180.
Lanier, L. M. & Volkman, L. E. 1998. Actin binding and nucleation.by Autographa california M nucleopolyhedrovirus. Virology, 243(1): 167-177.
Lawrence, M. S., Foellmer, H. G., Elsworth, J. D., Kim, J. H., Leranth, C., Kozlowski, D. A., Bothwell, A. L., Davidson, B. L., Bohn, M. C., & Redmond, D. E., Jr. 1999. Inflammatory responses and their impact on beta-galactosidase transgene expression following adenovirus vector delivery to the primate caudate nucleus. Gene Ther., 6(8): 1368-1379.
Lehtolainen, P., Tyynela, K., Kannasto, J., Airenne, K. J., & Yla-Herttuala, S. 2002. Baculoviruses exhibit restricted cell type specificity in rat brain: a comparison of baculovirus- and adenovirus-mediated intracerebral gene transfer in vivo. Gene Ther., 9(24): 1693-1699.
Ma, L, N Tamarina, Y Wang, A Kuznetsov, N Patel, C Kending, B J Hering, L H Philipson, (2000) Baculovirus-mediated gene transfer into pancreatic islet cells. Diabetes, 49: 1986-1991.
McLean, J. H., Shipley, M. T., & Bernstein, D. I. 1989. Golgi-like, transneuronal retrograde labelling with CNS injections of herpes simplex virus type 1. Brain Res.Bull., 22(5): 867-881.
McMenamin, M. M., Byrnes, A. P., Charlton, H. M., Coffin, R S., Latchman, D. S., & Wood, M. J. 1998. A gamma34.5 mutant of herpes simplex 1 causes severe inflammation in the brain. Neuroscience, 83(4): 1225-1237.
Millhouse, O. E. 1977. Optic chiasm collaterals afferent to the suprachiasmatic nucleus. Brain Res., 137(2): 351-355.
Mittoux, V., Ouary, S., Monville, C., Lisovoski, F., Poyot, T., Conde, F., Escartin, C., Robichon, R, Brouillet, E., Peschanski, M., & Hantraye, P. 2002. Corticostriatopallidal neuroprotection by adenovirus-mediated ciliary neurotrophic factor gene transfer in a rat model of progressive striatal degeneration. J.Neurosci., 22(11): 4478-4486.
Ni, YH, (2001) In vivo hepatic gene therapy. Acta Paediatr. Taiwan., 42 :191-200.
Norgren, R. B., Jr. & Lehman, M. N. 1998. Herpes simplex virus as a transneuronal tracer. Neurosci.Biobehav.Rev., 22(6): 695-708.
Norgren, R B., Jr., McLean, J. H., Bubel, H. C., Wander, A., Bernstein, D. I., & Lehman, M. N. 1992. Anterograde transport of HSV-1 and HSV-2 in the visual system. Brain Res.Bull., 28(3): 393-399.
O'Reilly, D. R., Miller, L. K., & Luchow, V. A. 1992. Baculovirus Expression Vectors: A Laboratory Manual. New York: W.H. Freeman and Company.
Peltekian, E., Garcia, L., & Danos, O. 2002. Neurotropism and retrograde axonal transport of a canine adenoviral vector: a tool for targeting key structures undergoing neurodegenerative processes. Mol.Ther., 5(1): 25-32.
Peters A. 1985. In A.Peters & E.G.Jones (Eds.), Cerebral Cortex: 19-80. New York: Plenum Press.
Pieroni, L, D Maione, N La Monica, (2001) In vivo gene transfer in mouse skeletal muscle mediated by baculovirus vectors. Hum. Gene Therp., 12: 871-881.
Raux, H., Flamand, A., & Blondel, D. 2000. Interaction of the rabies virus P protein with the LC8 dynein light chain. J.Virol., 74(21): 10212-10216.
Sandig, V, C Hofmann, S Steinert, G Jennings, P Schlag, M Strauss, (1996) Gene transfer into hepatocytes and human liver tissue by baculovirus vectors. Hum. Gene Ther., 7 :1937-1945.
Sarkis, C., Serguera, C., Petres, S., Buchet, D., Ridet, J. L., Edelman, L., & Mallet, J. 2000. Efficient transduction of neural cells in vitro and in vivo by a baculovirus-derived vector. Proc.Natl.Acad.Sci.U.S.A, 97(26): 14638-14643.
Sasahara, M, J W Fries, E W Raines, A M Gown, L E Westrum, M P Frosch, D T Bonthron, R Ross, T Collins, (1991) PDGF B-chain in neurons of the central nervous system, posterior pituitary, and in a trangenic model. Cell, 64: 217-227.
Soudais, C., Laplace-Builhe, C., Kissa, K., & Kremer, E. J. 2001. Preferential transduction of neurons by canine adenovirus vectors and their efficient retrograde transport in vivo. FASEB J., 15(12): 2283-2285.
Sun, N., Cassell, M. D., & Perlman, S. 1996. Anterograde, transneuronal transport of herpes simplex virus type 1 strain H129 in the murine visual system. J.Virol., 70(8): 5405-5413.
van Loo, N. D., Fortunati, E., Ehlert, E., Rabelink, M., Grosveld, F., & Scholte, B. J. 2001. Baculovirus infection of nondividing mammalian cells: mechanisms of entry and nuclear transport of capsids. J.Virol., 75(2): 961-970.
Vann, V. R & Atherton, S. S. 1991. Neural spread of herpes simplex virus after anterior chamber inoculation. Invest Ophthalmol.Vis.Sci., 32(9): 2462-2472.
Vercelli, A., Repici, M., Garbossa, D., & Grimaldi, A. 2000. Recent techniques for tracing pathways in the central nervous system of developing and adult mammals. Brain Res.Bull., 51(1): 11-28.
Wang, S., Ma, N., Gao, S. J., Yu, H., & Leong, K. W. 2001. Transgene expression in the brain stem effected by intramuscular injection of polyethylenimine/DNA complexes. Mol.Ther., 3(5 Pt 1): 658-664.

## Claims

1. Use of a baculoviral vector in the manufacture of a composition for treating a neuronal disorder,
wherein the vector is suitable for delivery to a target neuronal cell in a subject,
wherein the baculoviral vector is to be administered to a site in the striatum or the intra-vitreous body of the eye of the subject,
**characterised in that** the treatment is to be effected by the delivery of a nucleic acid by the baculoviral vector through axonal transport from the site of administration to the cell body of the target neuronal cell remote from the site of administration.

2. The use according to claim 1 wherein the nucleic acid is a therapeutic gene.

3. The use of any one of claims 1 or 2 wherein the vector comprises a coding sequence operably linked to a promoter.

4. The use of claim 3 wherein the promoter is a viral promoter.

5. The use of claim 4 wherein the promoter is CMV promoter.

6. The use of claim 3 wherein the promoter is a neuronal cell specific promoter.

7. The use of claim 6 wherein the neuronal cell specific promoter is operably linked to an enhancer.

8. The use of claim 7 wherein the enhancer is a heterologous enhancer that increases the neuronal cell specific transcriptional activity of the promoter.

9. The use of claim 8 wherein the neuronal cell specific promoter is PDGF β promoter and the enhancer is CMV E enhancer.

10. The use of any one of claims 1 to 9 wherein the vector is to be administered by injection.

11. The use of claim 10 wherein about 10⁶ to about 10⁹ pfu of the baculovirus is to be administered.

12. The use of any one of claims 1 to 11 wherein the axonal transport is by anterograde transport, retrograde transport or trans-synaptic transport.

13. The use of claim 12 wherein the neuronal disorder is stroke, ischemia, epilepsy, head and spinal cord trauma, Parkinson's disease, Huntington's disease, Alzheimer's disease, amyotrophic lateral sclerosis or neurogenetic disorder.

14. The use of claim 13 wherein the neuronal disorder is Parkinson's disease.

15. The use of claim 14 wherein the neuronal cell is a dopaminergic neuron in the substania nigra.

16. The use of claim 15 wherein the neuronal disorder is Huntington's disease.

17. The use of claim 16 wherein the neuronal cell is a corticostriatopallidal neurons.

18. A method for tracing an axonal neuronal pathway comprising detecting the transport pathway of a baculovirus adapted for detection that has been administered to a site in the central nervous system of a subject.

19. The method of claim 18 wherein the surface of the virus is labelled with a detectable marker.

20. The method of claim 19 wherein the detectable marker is Cy3.

21. The method of claim 20 wherein the baculovirus comprises a gene operably linked to a promoter, wherein the gene encodes a protein that can be detected.

22. The method of claim 21 wherein the promoter is a viral promoter.

23. The method of claim 22 wherein the viral promoter is CMV promoter.

24. The method of claim 23 wherein the promoter is a neuronal cell specific promoter.

25. The method of claim 24 wherein the neuronal cell specific promoter is operably linked to an enhancer.

26. The method of claim 25 wherein the neuronal specific promoter is PDGF β promoter and the enhancer is CMV E enhancer.

27. The method of claim 26 wherein the protein is a fluorescent protein.

28. The method of any one of claims 18 to 27 wherein the baculovirus has been administered by injection.

29. The method of claim 28 wherein the baculovirus has been administered by direct injection, stereotaxic injection or intra-vitreous injection.

30. The method of claim 29 wherein about 10⁶ to about 10⁹ pfu of the baculovirus has been administered.

31. The method of any one of claims 18 to 30 wherein the baculovirus is transported by anterograde transport, retrograde transport or trans-synaptic transport.

## Patentansprüche

1. Verwendung eines baculoviralen Vektors in der Herstellung einer Zusammensetzung zur Behandlung einer neuronalen Störung, wobei der Vektor zur Übertragung in eine neuronale Zielzelle in einem Subjekt geeignet ist, wobei der baculovirale Vektor an einen Ort im Striatum oder im Glaskörper des Auges des Subjekts zu verabreichen ist,
**dadurch gekennzeichnet, dass** die Behandlung durchzuführen ist durch die Übertragung einer Nukleinsäure durch den baculoviralen Vektor durch axonalen Transport vom Verabreichungsort auf den Zellkörper der neuronalen Zielzelle, die abseits vom Verabreichungsort ist.

2. Verwendung gemäß Anspruch 1, wobei die Nukleinsäure ein therapeutisches Gen ist.

3. Verwendung gemäß einem der Ansprüche 1 oder 2, wobei der Vektor eine kodierende Sequenz umfasst, die funktionsfähig an einen Promotor gekoppelt ist.

4. Verwendung gemäß Anspruch 3, wobei der Promotor ein viraler Promotor ist.

5. Verwendung gemäß Anspruch 4, wobei der Promotor ein CMV-Promotor ist.

6. Verwendung gemäß Anspruch 3, wobei der Promotor ein für neuronale Zellen spezifischer Promotor ist.

7. Verwendung gemäß Anspruch 6, wobei der für neuronale Zellen spezifische Promotor funktionsfähig an einen Verstärker gekoppelt ist.

8. Verwendung gemäß Anspruch 7, wobei der Verstärker ein heterologer Verstärker ist, der die für neuronale Zellen spezifische Transkriptionsaktivität des Promotors steigert.

9. Verwendung gemäß Anspruch 8, wobei der für neuronale Zellen spezifische Promotor der PDGFβ-Promotor ist und der Verstärker der CMV E-Verstärker ist.

10. Verwendung gemäß einem der Ansprüche 1 bis 9, wobei der Vektor durch Injektion zu verabreichen ist.

11. Verwendung gemäß Anspruch 10, wobei etwa 10⁶ bis etwa 10⁹ pfu des Baculovirus zu verabreichen sind.

12. Verwendung gemäß einem der Ansprüche 1 bis 11, wobei der axonale Transport durch anterograden Transport, retrograden Transport oder trans-synaptischen Transport erfolgt.

13. Verwendung gemäß Anspruch 12, wobei die neuronale Störung Schlaganfall, Ischämie, Epilepsie, Kopf- und Rückenmarkstrauma, Parkinson-Krankheit, Huntigton-Krankheit, Alzheimer-Krankheit, amyotrophe Lateralsklerose oder neurogenetische Störung ist.

14. Verwendung gemäß Anspruch 13, wobei die neuronale Störung Parkinson-Krankheit ist.

15. Verwendung gemäß Anspruch 14, wobei die neuronale Zelle ein dopaminerges Neuron in der Substantia nigra ist.

16. Verwendung gemäß Anspruch 15, wobei die neuronale Störung Huntington-Krankheit ist.

17. Verwendung gemäß Anspruch 16, wobei die neuronale Zelle ein corticostriatopallidales Neuron ist.

18. Verfahren zur Verfolgung eines axonalen neuronalen Weges, umfassend die Detektion des Transportweges eines zur Detektion angepassten Baculovirus, das an einen Ort im zentralen Nervensystem eines Subjekts verabreicht wurde.

19. Verfahren gemäß Anspruch 18, wobei die Oberfläche des Virus mit einem detektierbaren Marker markiert ist.

20. Verfahren gemäß Anspruch 19, wobei der detektierbare Marker Cy3 ist.

21. Verfahren gemäß Anspruch 20, wobei das Baculovirus ein Gen umfasst, das funktionsfähig an einen Promotor gekoppelt ist, wobei das Gen ein Protein kodiert, das detektiert werden kann.

22. Verfahren gemäß Anspruch 21, wobei der Promotor ein viraler Promotor ist.

23. Verfahren gemäß Anspruch 22, wobei der virale Promotor der CMV-Promotor ist.

24. Verfahren gemäß Anspruch 23, wobei der Promotor ein für neuronale Zellen spezifischer Promotor ist.

25. Verfahren gemäß Anspruch 24, wobei der für neuronale Zellen spezifische Promotor funktionsfähig an einen Verstärker gekoppelt ist.

26. Verfahren gemäß Anspruch 25, wobei der neuronalspezifische Promotor der PDGFβ-Promotor ist und der Verstärker der CMV E-Verstärker ist.

27. Verfahren gemäß Anspruch 26, wobei das Protein ein fluoreszentes Protein ist.

28. Verfahren gemäß einem der Ansprüche 18 bis 27, wobei das Baculovirus durch Injektion verabreicht wurde.

29. Verfahren gemäß Anspruch 28, wobei das Baculovirus durch direkte Injektion, stereotaktische Injektion oder intra-vitreale Injektion verabreicht wurde.

30. Verfahren gemäß Anspruch 29, wobei etwa 10⁶ bis etwa 10⁹ pfu des Baculovirus verabreicht wurden.

31. Verfahren gemäß einem der Ansprüche 18 bis 30, wobei das Baculovirus durch anterograden Transport, retrograden Transport oder trans-synaptischen Transport transportiert wird.

## Revendications

1. Utilisation d'un vecteur baculoviral dans la préparation d'une composition destinée à traiter un trouble neuronal, où le vecteur est approprié pour l'administration à une cellule neuronale cible chez un sujet, où le vecteur baculoviral doit être administré à un site dans le striatum ou le corps intravitreux de l'oeil du sujet, **caractérisé en ce que** le traitement doit être réalisé par l'administration d'un acide nucléique par le vecteur baculoviral par transport axonal à partir du site d'administration jusqu'au corps de cellule de la cellule neuronale cible éloignée du site d'administration.

2. Utilisation selon la revendication 1, où l'acide nucléique est un gène thérapeutique.

3. Utilisation selon l'une quelconque des revendications 1 ou 2, où le vecteur comprend une séquence codante liée de manière fonctionnelle à un promoteur.

4. Utilisation selon la revendication 3, où le promoteur est un promoteur viral.

5. Utilisation selon la revendication 4, où le promoteur est un promoteur CMV.

6. Utilisation selon la revendication 3, où le promoteur est un promoteur spécifique des cellules neuronales.

7. Utilisation selon la revendication 6, où le promoteur spécifique des cellules neuronales est lié de manière fonctionnelle à un activateur.

8. Utilisation selon la revendication 7, où l'activateur est un activateur hétérologue qui augmente l'activité transcriptionnelle spécifique des cellules neuronales du promoteur.

9. Utilisation selon la revendication 8, où le promoteur spécifique des cellules neuronales est le promoteur PDGF β et l'activateur est l'activateur CMV E.

10. Utilisation selon l'une quelconque des revendications 1 à 9, où le vecteur doit être administré par injection.

11. Utilisation selon la revendication 10, où environ 10⁶ à environ 10⁹ pfu du baculovirus doivent être administrées.

12. Utilisation selon l'une quelconque des revendications 1 à 11, où le transport axonal est effectué par transport antérograde, transport rétrograde ou transport trans-synaptique.

13. Utilisation selon la revendication 12, où le trouble neuronal est un accident vasculaire, l'ischémie, l'épilepsie, un traumatisme de la tête et de la moelle épinière, la maladie de Parkinson, la maladie de Huntington., la maladie d'Alzheimer, la sclérose latérale amyotrophique ou un trouble neurogénétique.

14. Utilisation selon la revendication 13, où le trouble neuronal est la maladie de Parkinson.

15. Utilisation selon la revendication 14, où la cellule neuronale est un neurone dopaminergique dans la substance noire.

16. Utilisation selon la revendication 15, où le trouble neuronal est la maladie de Huntington.

17. Utilisation selon la revendication 16, où la cellule neuronale est un neurone corticostriatopallidal.

18. Procédé pour dépister une voie neuronale axonale consistant à détecter la voie de transport d'un baculovirus adapté pour la détection qui a été administré à un site dans le système nerveux central d'un sujet.

19. Procédé selon la revendication 18, où la surface du virus est marquée avec un marqueur détectable.

20. Procédé selon la revendication 19, où le marqueur détectable est Cy3.

21. Procédé selon la revendication 20, où le baculovirus comprend un gène lié de manière fonctionnelle à un promoteur, lequel gène code pour une protéine qui peut être détectée.

22. Procédé selon la revendication 21, où le promoteur est un promoteur viral.

23. Procédé selon la revendication 22, où le promoteur viral est un promoteur CMV.

24. Procédé selon la revendication 23, où le promoteur est un promoteur spécifique des cellules neuronales.

25. Procédé selon la revendication 24, où le promoteur spécifique des cellules neuronales est lié de manière fonctionnelle à un activateur.

26. Procédé selon la revendication 25, où le promoteur spécifique des cellules neuronales est le promoteur PDGF β et l'activateur est l'activateur CMV E.

27. Procédé selon la revendication 26, où la protéine est une protéine fluorescente.

28. Procédé selon l'une quelconque des revendications 18 à 27, où le baculovirus a été administré par injection.

29. Procédé selon la revendication 28, où le baculovirus a été administré par injection directe, injection stéréotaxique ou injection intravitreuse.

30. Procédé selon la revendication 29, où environ 10⁶ à environ 10⁹ pfu du baculovirus ont été administrées.

31. Procédé selon l'une quelconque des revendications 18 à 30, où le baculovirus est transporté par transport antérograde, transport rétrograde ou transport trans-synaptique.


### SEQUENCE LISTING

<110> Wang, Shu
   Li, Ying
<120> METHOD FOR GENE DELIVERY TO NEURONAL CELLS
<130>
<160> 2
<170> PatentIn version 3.2
<210> 1 ,
   <211> 20
   <212> DNA
   <213> artificial
<220>
   <223> synthetic primer
<400> 1
   attgctcaac agtatgggca 20
<210> 2
   <211> 21
   <212> DNA
   <213> artificial
<220>
   <223> synthetic primer
<400> 2
   cgaagaagga gaatagggtt g 21